# EUROPÄISCHE PATENTSCHRIFT

(11) **EP 1 495 744 B1**
(45) Veröffentlichungstag und Bekanntmachung des Hinweises auf die Patenterteilung: **18.01.2006**
(21) Anmeldenummer: 03015418.1
(22) Anmeldetag: 09.07.2003
(51) Int. Cl.: A61G 13/00

(54) **Einrichtung zur Versorgung eines Operationsraumes mit kontaminationsfreier Luft, Energie und sonstigen Versorgungsmedien**
Installation for supplying clean air, power and other medical supplies to a surgical room
Installation pour fournir de l'air purifié, de l'énergie et d'autres produits médicaux à une salle d'opération

(43) Veröffentlichungstag der Anmeldung: 12.01.2005
(73) Patentinhaber: van den Wildenberg, Pierre, 6330 Cham-Hüneberg (CH)
(72) Erfinder: van den Wildenberg, Pierre, 6330 Cham-Hüneberg (CH)
(74) Vertreter: Fleck, Thomas

(56) Entgegenhaltungen:
- EP-A- 0 943 306
- DE-A- 2 512 679
- US-A- 3 380 369
- US-A- 4 164 173

## Beschreibung

Die Erfindung betrifft eine Einrichtung zur Versorgung eines Operationsraumes mit kontaminationsfreier Luft, Energie und sonstigen Versorgungsmedien.

Einrichtungen zur Versorgung eines Operationsraumes (OP) mit kontaminationsfreier Luft, Energie und sonstigen Versorgungsmedien sind bekannt. Siehe DE 2512679 A, die als Basis für die Formulierung des Oberbegriffs des Anspruchs 1 herangezogen worden ist.

Insbesondere im sogenannten Kernbereich eines OP, dem Bereich, in dem die offene Wunde liegt, ist ein Höchstmaß an Sterilität erforderlich. Daher ist es bekannt, wenigstens diesen Bereich mit kontaminationsfreier Luft, das ist Luft, deren Keim- und Erregergehalt bspw. durch Filtern auf ein Minimalmaß abgesenkt wurde, zu versorgen. Hierfür werden Luftauslässe, aus denen die kontaminationsfreie Luft ausströmt, verwendet. Solche Luftauslässe sind als solche in verschiedenen Querschnittsformen, auch mit oktogonalem Querschnitt bekannt.

Weiter ist es bekannt, in einem OP Energieanschlüsse und sonstige Anschlüsse für Versorgungsmedien (Gasanschlüsse, Datenanschlüsse für die EDV etc.) in den Bereich, in dem operiert wird, über entsprechende Medienbrücken oder ähnliches über Kopf zuzuführen. Dabei müssen für eine Operation spezifische Anschlüsse sowohl für einen Chirurgen als auch für einen Anästhesisten bereitgestellt werden.

Schließlich ist bekannt, daß das während der Operation benötigte Arbeitsmaterial (bspw. das Chirurgenbesteck) auf auf Rollen fahrbaren Zubehörwagen bereitgehalten und für die Operation in den OP an den jeweiligen Arbeitsplatz gebracht wird. Gleiches gilt für fahr- bzw. tragbare Geräte wie Herz-Lungen-Maschine, Sondenmonitore etc. Diese Geräte bzw. Zubehörwagen stehen dann während der Operation auf dem Boden des OP und behindern nicht selten die Bewegungsfreiheit des an der Operation beteiligten Klinkpersonals. Dies kann in kritischen Situationen während einer Operation nachteilig sein und sogar zu einer Gefährdung des Patienten führen.

Es ist **Aufgabe** der Erfindung, eine solche Einrichtung der eingangs genannten Art anzugeben, die ein hohes Maß an Flexibilität bei der Einrichtung des Operationsraumes für die jeweils anstehende Operation sowie ein Arbeiten mit einem hohen Maß an Bewegungsfreiheit während der Operation ermöglicht.

Diese Aufgabe wird erfindungsgemäß durch eine Einrichtung gemäß Anspruch 1 **gelöst.**

Der oktogonale Luftauslaß deckt einen hochsterilen Bereich des OP ab, in dem während der Operation wenigstens der Wundbereich des Patienten angeordnet ist und in den stets nach Art einer Luftdusche kontaminationsfreie Luft eingeblasen wird.

Unmittelbar angrenzend an den Luftauslaß sind an jeweils eine Seite des oktogonalen Luftauslasses zwischen sich lassenden Seiten von einer Decke herabhängende Versorgungssäulen angeordnet, die die Versorgungsanschlüsse tragen. Damit können je nach Arbeitsplatzaufteilung bei der bevorstehenden Operation die Anschlüsse der jeweils passenden Versorgungssäulen genutzt werden, was den Einsatz kurzer Versorgungsschläuche und -kabel erlaubt. Diese sind während der Operation weniger störend.

Die Versorgungssäulen sind höhenverstellbar, was eine individuelle Einstellung der gewünschten Anschlußhöhe durch die die jeweilige Versorgungssäule nutzende Person gestattet. Außerdem erlaubt die Höhenverstellbarkeit der Versorgungssäulen in Verbindung mit der Kupplungsvorrichtung ein Anheben eines angekuppelten Geräts/Zubehörwagens, um dieses/diesen aus dem Bodenbereich zu entfernen. Dies schafft zum einen eine verbesserte Bodenfreiheit und damit mehr Bewegungsspielraum für das während der Operation arbeitende Klinikpersonal, erlaubt zu anderen ein Anheben der Geräte/Zubehörwagen auf eine angenehme Arbeitshöhe.

Durch die oktogonale Querschnittsform des Luftauslasses und die Anordnung der Versorgungssäulen an jeder zweiten Seite des Luftauslasses wird durch die Versorgungssäulen ein Viereck, bei einem bevorzugt verwendeten Luftauslaß mit einem Querschnitt von regelmäßig oktogonaler Form ein Quadrat, um das Zentrum des Luftaulasses, der zugleich das Zentrum des Arbeitsbereiches während der Operation markiert, herum gebildet. Diese Anordnung erlaubt eine hohe Flexibilität bei der individuellen Verteilung der Arbeitsplätze für jede spezielle Operation.

Weitere vorteilhafte Ausgestaltungen ergeben sich aus den abhängigen Ansprüchen 2 bis 8.

Sind, wie gemäß Anspruch 2 vorgesehen, die vier Versorgungssäulen identisch mit Versorgungsanschlüssen versehen, wird die Flexibilität bei der Verteilung der Arbeitspositionen für die Operation noch einmal erhöht. Bei einer Ausgestaltung gemäß Anspruch 3 kann entsprechend an jeder der vier Säulen alternativ ein Chirurg (Energieversorgung, Absaugung etc.) oder ein Anästhesist (Narkosegas etc.) eingesetzt werden.

Die in Anspruch 4 beschriebene Anordnung der Versorgungsanschlüsse in um die Versorgungssäule in ihrem unteren Ende umlaufenden, geneigten Anschlußleisten erleichtert die Zugänglichkeit der Anschlüsse. Sie sind auch während einer laufenden Operation erforderlichenfalls schnell und sicher erreichbar.

Versorgungssäulen mit einem rechteckigen, vorzugsweise quadratischen Querschnitt (Anspruch 5) haben sich als geeignet herausgestellt, da die Anschlüsse auf geraden Abschnitten angebracht, jedoch gut auf engem Raum um die Säule herum verteil werden können.

Eine besonders symmetrische und damit vielseitige Anordnung der Versorgungssäulen ist in Anspruch 6 angegeben. Die Ausrichtung der Versorgungssäulen mit die benachbarten Seiten des Luftauslasses unter rechtem Winkel schneidenden Diagonalen führt zu einer guten Zugänglichkeit von zwei der vier Seiten der Säule von dem sterilen Bereich innerhalb des Luftauslasses her.

Eine Anordnung wie in Anspruch 7 beschrieben führt zu einem insgesamt kompakten Aufbau der Einrichtung und damit auch zu einem optisch ansprechenden Erscheinungsbild.

Sind die Kupplungsvorrichtungen wie in Anspruch 8 beschrieben ausgebildet, kann durch Drehen des an der Versorgungssäule angekoppelten, frei hängenden Gerätes/Zubehörwagens dieses/dieser in die gerade benötigte Position geacht werden, was wiederum die Bewegungsfreiheit erhöht und das Arbeiten insgesamt erleichtert. Um ein Zurückdrehen zu verhindern, können Rastmittel vorhanden sein, oder es kann eine Reibungsdämpfung bzw. eine andere Dämpfung vorgesehen sein.

Weitere Merkmale und Vorteile der Erfindung ergeben sich aus dem nachfolgend anhand der Zeichnung beschriebenen Ausführungsbeispiel. In der Zeichnung zeigen:
- Fig. 1: eine Ansicht einer an einer Decke eines Operationsraumes angeordneten, erfindungsgemäßen Einrichtung,
- Fig. 2: die Anschlußleisten an einer Versorgungssäule in einer Ansicht von unten sowie Ansichten von den vier Seiten und
- Fig. 3: eine Versorgungssäule der erfindungsgemäßen Einrichtung mit angekoppeltem Zubehörwagen in einer angehobenen Betriebsposition (links) und einer abgesenkten Position (rechts) zum Ankoppeln bzw. Lösen des Zubehörwagens.

In den Figuren sind gleiche Teile mit gleichen Bezugszeichen versehen.

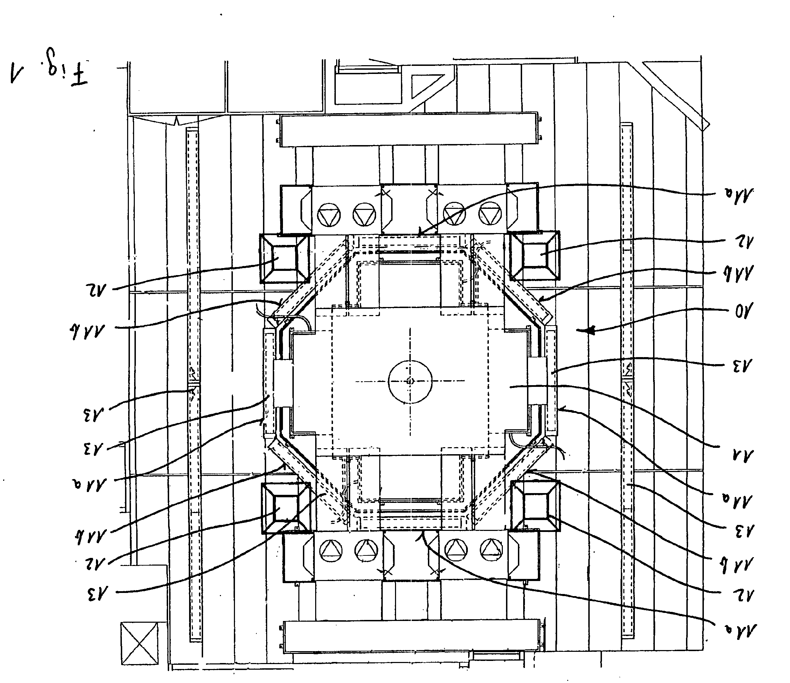

In Fig. 1 ist eine erfindungsgemäße Einrichtung 10 zur Versorgung eines Operationsraumes mit kontaminationsfreier Luft, Energie und anderen Versorgungsmedien in einer Ansicht von unten gezeigt. Die Einrichtung 10 ist an einer Decke eines Operationsraumes aufgehängt und bietet darunter Bodenfreiheit. In ihrem Zentrum ist ein Luftauslaß 11 angeordnet, der einen regelmäßigen, oktogonalen Querschnitt aufweist. An vier jeweils durch eine weitere Seite 11a des Luftauslasses 11 getrennten Seiten 11b angrenzend sind jeweils von der Decke herabhängende Versorgungssäulen 12 angeordnet, die die Versorgungsanschlüsse für Energie und andere Versorgungsmedien tragen und ebenfalls Bestandteil der Einrichtung 10 sind. Die Versorgungssäulen 12 sind vertikal höhenverstellbar, d. h. sie können angehoben und abgesenkt werden: Dies ist in diesem Ausführungsbeispiel für jede Versorgungssäule 12 individuell möglich. Die vier Versorgungssäulen 12 sind an den Eckpunkten eines gedachten Quadrates angeordnet, und zwar derart, daß ihre eine Diagonale die angrenzende Seite 11b des Luftauslasses 11 in deren Mittelpunkt senkrecht schneidet. So sind die einzelnen Versorgungssäulen 12 bezogen auf den Mittelpunkt des Luftauslasses 11 jeweils um 90° versetzt angeordnet. Die außenliegenden Kanten der Versorgungssäulen 12 liegen zu den zwischen den Säulen gelegenen Seiten 11a des Luftauslasses 11 parallel und fluchten in etwa mit diesen. Auf diese Weise ergibt sich insgesamt eine Einrichtung 10 mit einer kompakten, quadratisch anmutenden Außenform. Alle Versorgungssäulen 12 sind gleichartig aufgebaut und jeweils mit Versorgungsanschlüssen für die Arbeitsplätze eines Chirurgen sowie eines Anästhesisten bestückt. Zudem können weitere Versorgungsanschlüsse vorgesehen sein (z. B. Datenanschlüsse). Im Bereich der Einrichtung 10 ist ebenfalls die Operationsraumbeleuchtung mit Leuchten 13 vorgesehen, von denen jeweils eine an den acht Seiten des Luftauslasses 11 angeordnet ist sowie weitere außerhalb der eigentlichen Einrichtung 10.

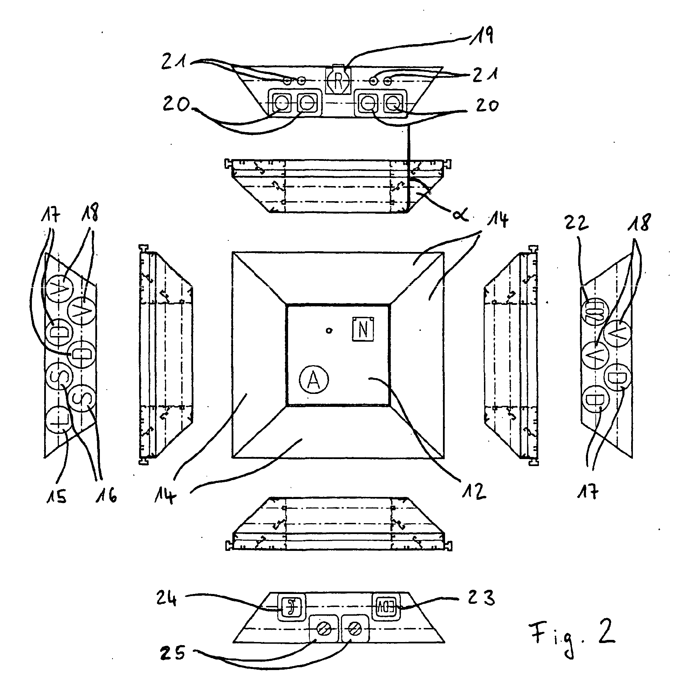

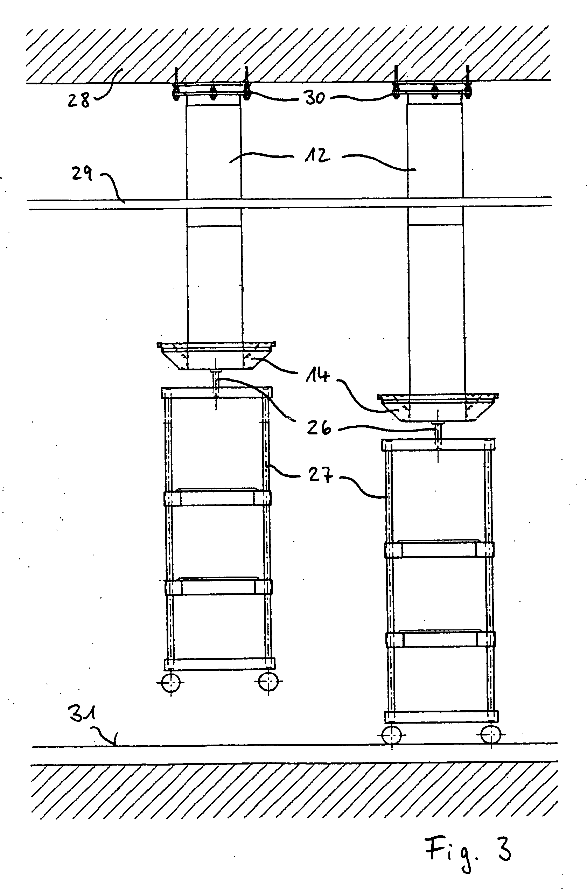

Die Versorgungssäulen 12 sind in den Figuren 2 und 3 näher dargestellt und werden anhand dieser eingehender erläutert.

In Figur 2 ist in verschiedenen Ansichten der untere Bereich einer Versorgungssäule 12 zu erkennen mit daran angeordneten Versorgungsleisten 14. An den Versorgungsleisten 14 sind Versorgungsanschlüsse 15 - 25 für die Versorgung mit Energie und den verschiedensten Medien, wie bspw. Gasen aber auch Unterdruck für die Absaugung, Daten etc., angeordnet. Diese sind wegen der Neigung der Anschlußleisten 14 zur Vertikalen um den Winkel α von 45° leicht zugänglich.

In Fig. 3 ist eine Versorgungssäule 12 gezeigt, wie sie über eine Kupplungsvorrichtung 26 mit einem auf Rollen fahrbaren Zubehörwagen 27 verbunden ist. In der linken Darstellung ist die Versorgungssäule 12 in einer Betriebsposition, deren Höhe variabel und individuell einstellbar ist und in der der Zubehörwagen 27 frei über dem Boden 31 schwebt, gezeigt, wohingegen die rechte Darstellung die abgesenkte Position zum An- bzw. Abkuppeln des Zubehörwagens 27 zeigt. Der angehobene Zubehörwagen 27 ist im gezeigten Beispiel aufgrund der Ausbildung der Kupplungsvorrichtung 26 um 330° um die Längsachse der Versorgungssäule 12 drehbar. Während einer Operation nicht benötigte Versorgungssäulen 12 können auf die maximale Höhe nach oben verschoben werden, um noch weitere Bewegungsfreiheit im Operationsraum zu erhalten.

In Figur 3 ist auch die für einen Operationsraum typische Situation dargestellt, wonach die Versorgungssäule 12 an einer Rohdecke 28 aufgehängt ist und durch eine Zwischendecke 29 hindurchragt, die den eigentlichen Abschluß des Operationsraumes nach oben bildet. Die Versorgungssäule 12 wird dabei von einem fernsteuerbaren Verfahrantrieb 30 angehoben bzw. abgesenkt.

Schließlich kann in Figur 3 erkannt werden, daß die Versorgungsleisten 14 über die Außenabmessungen der sonstigen Versorgungssäule 12 überstehen und so einen Abschluß in der Form eines umgedrehten Pilzes ergeben.

Mit der Erfindung wird ein multidisziplinär nutzbarer, servicefreundlicher, preiswerter und ergonomischer Operationsraum geschaffen, der völlige Positionsfreiheit des Operationstisches erlaubt und trotzdem kurze Verbindungsschläuche und Elektrokabel für jeden Einsatz gewährleistet. Der Operationsraum kann für jeden Operationseinsatz individuell und vielseitig genutzt und mit Arbeitsplätzen belegt werden, ohne daß hierfür komplizierte Umbauten erforderlich sind. Die Erfindung ist nicht auf das zur Erläuterung gezeigte Ausführungsbeispiel beschränkt, sondern wird durch die nachfolgenden Ansprüche in ihrer vollständigen Breite definiert.

### Bezugszeichenliste

- 10: Einrichtung
- 11: Luftauslaß
- 11a: Seite
- 11b: Seite
- 12: Versorgungssäule
- 13: Leuchte
- 14: Versorgungsleiste
- 15 - 25: Versorgungsanschluß
- 26: Kupplungsvorrichtung
- 27: Zubehörwagen
- 28: Rohdecke
- 29: Zwischendecke
- 30: Verfahrantrieb
- 31: Boden

- α: Winkel

## Patentansprüche

1. Einrichtung zur Versorgung eines Operationsraumes mit kontaminationsfreier Luft, Energie und sonstigen Versorgungsmedien, die an einer Decke (28) des Operationsraumes aufhängbar ist und einen Luftauslaß (11) und um den Luftauslaß (11) herum angeordnete Versorgungssäulen (12) mit Versorgungsanschlüssen (15 - 25) zur Versorgung mit Energie und/oder sonstigen Versorgungsmedien aufweist, wobei die Versorgungssäulen (12) höhenverstellbar sind, **dadurch gekennzeichnet, daß** der Luftauslaß (11) eine oktogonale Querschnittsform aufweist und **daß** die Versorgungssäulen (12) an jeweils durch eine weitere Seite (11a) des Oktogons getrennten Seiten (11b) des Luftauslasses (11) angeordnet sind, und von einer Decke (28) des Operationsraumes höhenverstellbar herabhängbar sind, wobei an den Versorgungssäulen (12) die Versorgungsanschlüsse (15-25) angeordnet sind und wobei die Versorgungssäulen (12) Kupplungsvorrichtungen (26) zum Verbinden mit fahr- und/oder tragbaren Geräten und/oder Zubehörwagen (27) aufweisen.

2. Einrichtung nach Anspruch 1, **dadurch gekennzeichnet, daß** die vier Versorgungssäulen (12) identisch mit Versorgungsanschlüssen (15-25) versehen sind.

3. Einrichtung nach Anspruch 2, **dadurch gekennzeichnet, daß** jede Versorgungssäule (12) Versorgungsanschlüsse (15-25) für die Versorgung eines Chirurgen- wie eines Anästhesiearbeitsplatzes aufweist.

4. Einrichtung nach einem der vorhergehenden Ansprüche, **dadurch gekennzeichnet, daß** die Versorgungssäulen (12) an ihren unteren, dem Boden (31) des Operationsraumes zugewandten Enden umlaufende, nach außen vorstehende, um einen Winkel (α) von 30° bis 60°, vorzugsweise 45° gegenüber der Vertikalen geneigte Anschlußleisten (14) aufweisen, in denen die Versorgungsanschlüsse (15-25) angeordnet sind.

5. Einrichtung nach einem der vorhergehenden Ansprüche, **dadurch gekennzeichnet, daß** die Versorgungssäulen (12) einen rechteckigen, vorzugsweise quadratischen Querschnitt aufweisen.

6. Einrichtung nach Anspruch 5, **dadurch gekennzeichnet, daß** die Versorgungssäulen (12) so um jeweils 90° versetzt zu dem Luftauslaß (11) ausgerichtet sind, daß eine Diagonale des Querschnitts der Versorgungssäule (12) senkrecht auf der der Versorgungssäule (12) benachbarten Seite (11b) des oktogonalen Querschnitts des Lufteinlasses (11) steht und der Schnittpunkt dieser Diagonalen und der Seite im Mittelpunkt der Seite (11b) liegt.

7. Einrichtung nach Anspruch 6, **dadurch gekennzeichnet, daß** die dem Zentrum des Luftauslasses (11) abgewandten Außenkanten der Versorgungssäulen (12) mit den Seiten (11a) des Luftauslasses (11), an denen keine Versorgungssäulen (12) anliegen, in etwa fluchten.

8. Einrichtung nach einem der vorhergehenden Ansprüche, **dadurch gekennzeichnet, daß** die Kupplungsvorrichtung (26) so ausgebildet ist, daß ein eingehängtes Gerät / ein eingehängter Zubehörwagen (27) um wenigstens 180°, vorzugsweise um 330° um die Längsachse der Versorgungssäule (12) drehbar ist.

## Claims

1. Installation for supplying an operating theatre with uncontaminated air, power and other supply media which can be suspended on a ceiling (28) of the operating theatre and with supply columns (12) having supply connections (15-25) arranged around the air outlet (11) for supplying with power and/or other supply media, the supply columns (12) being vertically adjustable,
**characterized in that** the air outlet (11) has an octagonal cross-sectional shape and that the supply columns (12) are located at sides (11b) of the air outlet (11) in each case separated by a further side (11a) of the octagon and can be hung down in vertically adjustable manner from a ceiling (28) of the operating theatre, the supply connections (15-25) being located on the supply columns (12), which have coupling devices (26) for connection to mobile and/or portable equipment and/or accessories trolleys (27).

2. Installation according to claim 1, **characterized in that** the four supply columns (12) are identically provided with supply connections (15-25).

3. Installation according to claim 2, **characterized in that** each supply column (12) has supply connections (15-25) for the supply of a surgeon, such as an anesthetist workplace.

4. Installation according to one of the preceding claims, **characterized in that** the lower ends of the supply columns (12) facing the operating theatre floor (31) have circumferential, outwardly projecting connection strips (14) inclined relative to the vertical by an angle (α) of 30 to 60°, preferably 45° and in which are located the supply connections (15-25).

5. Installation according to one of the preceding claim, **characterized in that** the supply columns (12) have a rectangular, preferably square cross-section.

6. Installation according to claim 5, **characterized in that** the supply columns (12) are so oriented displaced in each case by 90° to the air outlet (11) that a diagonal of the cross-section of the supply column (12) is perpendicular on the side (11b) of the octagonal cross-section of the air outlet (11) adjacent to the supply column (12) and the intersection point of said diagonal and the side is at the midpoint of the side (11b).

7. Installation according to claim 6, **characterized in that** the outer edges of the supply columns (12) remote from the centre of the air outlet (11) are roughly aligned with the sides (11a) of the air outlet (11) against which no supply columns (12) bear.

8. Installation according to one of the preceding claims, **characterized in that** the coupling device (26) is constructed in such a way that a hung in equipment or accessories trolley (27) is rotatable by at least 180°, preferably by 330° about the longitudinal axis of the supply column (12).

## Revendications

1. Dispositif pour alimenter une salle d'opération en air non contaminé, en énergie et en autres fluides d'alimentation, qui peut être suspendu au plafond (28) de la salle d'opération et présente une sortie d'air (11) et des colonnes d'alimentation (12) munies de raccords d'alimentation (15 - 25) disposées autour de la sortie d'air (11) pour l'alimentation en énergie et/ou autres fluides d'alimentation, les colonnes d'alimentation (12) étant réglables en hauteur,
**caractérisé en ce que** la sortie d'air (11) a une forme de section transversale octogonale, et les colonnes d'alimentation (12) sont disposées sur des faces (11b) de la sortie d'air (11) respectivement séparées par une autre face (11a) de l'octogone et peuvent être suspendues réglables en hauteur sous un plafond (28) de la salle d'opération, les raccords d'alimentation (15-25) étant disposés sur les colonnes d'alimentation (12) et les colonnes d'alimentation (12) présentant des dispositifs de couplage (26) pour le raccordement d'appareils mobiles ou portables et/ou de chariots à accessoires (27).

2. Dispositif selon la revendication 1,
**caractérisé en ce que** les quatre colonnes d'alimentation (12) sont munies de manière identique de raccords d'alimentation (15-25).

3. Dispositif selon la revendication 2,
**caractérisé en ce que** chaque colonne d'alimentation (12) présente des raccords d'alimentation (15-25) pour alimenter un poste de travail d'un chirurgien ou d'un anesthésiste.

4. Dispositif selon l'une quelconque des revendications précédentes,
**caractérisé en ce que** les colonnes d'alimentation (12), au niveau de leurs extrémités inférieures tournées vers le sol (31) de la salle d'opération, présentent des baguettes de raccordement (14) périphériques saillant vers l'extérieur inclinées selon un angle (α) de 30° à 60°, de préférence 45°, par rapport à la verticale, dans lesquelles sont disposés les raccords d'alimentation (15-25).

5. Dispositif selon l'une quelconque des revendications précédentes,
**caractérisé en ce que** les colonnes d'alimentation (12) présentent une section transversale rectangulaire, de préférence carrée.

6. Dispositif selon la revendication 5,
**caractérisé en ce que** les colonnes d'alimentation (12) sont respectivement décalées de 90° par rapport à la sortie d'air (11) de telle sorte qu'une diagonale de la section transversale de la colonne d'alimentation (12) s'étend perpendiculairement à la face (11b) de la section transversale octogonale de la sortie d'air (11), voisine de la colonne d'alimentation (12), et le point d'intersection de cette diagonale et de la face se situe au centre de la face (11b).

7. Dispositif selon la revendication 6,
**caractérisé en ce que** les arêtes extérieures des colonnes d'alimentation (12), opposées au centre de la sortie d'air (11), sont sensiblement alignées avec les faces (11a) de la sortie d'air (11) auxquelles aucune colonne d'alimentation (12) n'est adossée.

8. Dispositif selon l'une quelconque des revendications précédentes,
**caractérisé en ce que** le dispositif de couplage (26) est conçu pour pouvoir faire tourner un appareil / un chariot à accessoires (27) accroché selon au moins 180°, de préférence 330°, autour de l'axe longitudinal de la colonne d'alimentation (12).
